# EUROPEAN PATENT SPECIFICATION

(11) **EP 2 296 772 B1**
(45) Date of publication and mention of the grant of the patent: **02.08.2017**
(21) Application number: 09793912.8
(22) Date of filing: 23.06.2009
(51) Int. Cl.: B01D 9/00, A61K 9/16, C07K 14/37

(54) **AMPHIPHILIC PROTEINS AS MORPHOLOGY MODIFIERS**
AMPHIPHILE PROTEINE ALS MORPHOLOGIE-MODIFIKATOREN
PROTÉINES AMPHIPHILES EN TANT QUE MODIFICATEURS DE LA MORPHOLOGIE

(30) Priority: 11.07.2008 EP 08160212
(43) Date of publication of application: 23.03.2011
(73) Proprietor: B.R.A.I.N. Biotechnology Research and Information Network AG, 64673 Zwingenberg (DE)
(72) Inventor: HAFNER, Andreas, CH-4460 Gelterkinden (CH); BUTHE, Andreas, 48565 Steinfurt (DE); VAN DER SCHAAF, Paul Adriaan, F-68220 Hagenthal-le-Haut (FR); KAUFMANN, Franz, 79106 Freiburg (DE); BRADLEY, Gordon, CH-4410 Liestal (CH)
(74) Representative: Vossius & Partner Patentanwälte Rechtsanwälte mbB
(86) International application number: PCT/EP2009/057760
(87) International publication number: WO 2010/003811

(56) References cited:
- WO-A-96/41882
- WO-A-2005/115344
- WO-A-2008/035962
- WO-A1-2008/142111
- US-A1- 2003 049 323
- US-A1- 2003 215 517
- SCHOLMEIJER K ET AL: "FUNGAL HYDROPHOBINS IN MEDICAL AND TECHNICAL APPLICATIONS", APPLIED MICROBIOLOGY AND BIOTECHNOLOGY, SPRINGER VERLAG, BERLIN, DE, vol. 56, no. 1/02, 1 July 2001 (2001-07-01), pages 1-08, XP001120015, ISSN: 0175-7598, DOI: 10.1007/S002530100632

## Description

The present invention relates to a process for modifying the morphology and/or polymorphism of solid organic compounds as characterized in the appended claims.

Solidification and especially crystallization is a key separation and purification unit in most of the pharmaceutical, food and specialty chemical processes (for example pigments), with a significant impact on the efficiency and profitability of the overall process. The majority of pharmaceutical products contain active ingredients produced in crystalline form. Thus crystallisation is of fundamental importance to the industry. For efficient downstream operation (such as filtration, drying, compacting) and product effectiveness (e.g. bioavailability, tablet stability) the control of crystal purity, size distribution and shape can be critically important.

Typically pharmaceutical-grade crystalline products require a narrow particle size distribution, which implies that the primary production process must be well-designed and tightly controlled under optimal conditions. Control of crystal size and shape enables the optimization of the dissolution rate and this may maximize the benefit while minimizing the side effects. Many pharmaceuticals can form crystals that exhibit multiple morphological forms and habits that are of critical importance to the formulation and end use properties of the products.

### Technical background

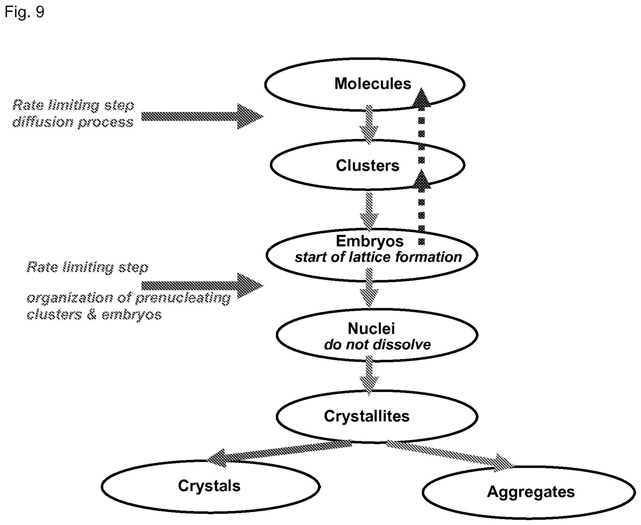

Figure 9 illustrates a typical crystallization process embracing the following steps:
(a) Organic molecules form randomly orientated molecular clusters by a diffusion process.
(b) These clusters can either break down to single molecules again or begin to form unstable lattice formations called embryos.
(c) Such embryos can break down into clusters again or grow sufficiently to form stable nuclei which precipitate out of solution (nucleation). Such critical size is dictated by the operating conditions (temperature, supersaturation, etc.).
(d) These nuclei then grow into larger crystallites which can continue to grow into single crystals or come together to form aggregates of crystallites.
(e) Such aggregates can range from soft aggregates that can be easily broken down to the original crystallites to hard aggregates that can only be broken down by an aggressive process such as milling.

Supersaturation is the driving force of the crystallization, hence the rate of nucleation and growth is driven by the existing supersaturation in the solution. Supersaturation is defined as concentration of the solute in excess of saturated concentration under given conditions of temperature. Once supersaturation is lost, the solid-liquid system reaches equilibrium and the crystallization process stops.

Nucleation and growth continue to occur simultaneously while the supersaturation exists.

Certain solvents, the presence of impurities or additives and compounds of similar chemical type to the compound undergoing the crystallization process can strongly influence its nucleation and crystal growth stages by changing the supersaturation properties of the crystallization process.

Depending upon the conditions, either nucleation or growth may be predominant over the other, and as a result, crystals with different sizes, different size distributions and habits (shapes) are obtained.

Crystal habits can be, for example, cubic, tetragonal, orthorhombic, hexagonal, monoclinic, triclinic, and trigonal.

Different polymorphs can also be produced by changes in the crystallization process.

Polymorphs are defined as crystalline phases that have different arrangements and/or conformations of molecules in the crystal lattice. These crystal forms differ in packing, thermodynamic, spectroscopic, kinetic, surface and mechanical properties.

Although polymorphs have the same elemental composition, polymorphs exhibit different physico-chemical and physicotechnical properties such as free energy, entropy, heat capacity, melting point, sublimation temperature, solubility, stability, dissolution rate, bioavailability, hardness, compatibility, flowability, tensile strength and compressibility, etc.

For this reason, polymorphism is of major importance in industrial manufacture of crystalline products

The nature of a crystallization process is governed by both thermodynamic and kinetic factors, which can make it highly variable and difficult to control. Factors such as impurity level, mixing regime, vessel design, and cooling profile can have a major impact on the size, number, and shape of crystals produced.

Poor control of crystal size and shape can also result in unacceptably long filtration or drying times, or in extra processing steps, such as recrystallization or milling, and can influence the purity of the product which is especially important in the food and pharmaceutical industries, in which the crystals are consumed.

It is known that the morphology and size of bio-active substances can be affected by the solvent used in the crystallization process. For example, monoclinic paracetamol is formed by crystallization from ethanol, but the less stable polymorph, orthorhombic paracetamol, is formed by slow crystallization from hot water only when multiple nucleation is prevented. However, the choice of crystallization solvents is severely limited on toxicity grounds.

A number of additives have already been used to influence either the growth or the nucleation phase, resulting in modification of either the polymorphic form or the crystal habit. In some cases, paracetamol serves here as a model substance.

Synthetic (co)polymers and surfactants have also been shown to modify the morphology of bio-active substances but this has limited commercial value again on toxicity grounds.

WO03/033462 proposes polymer libraries for initiating growth of crystal polymorphs and describes the use of certain polymers to modify the crystallization of paracetamol: The crystals are grown by cooling a solution of paracetamol in hot water. In the absence of polymers, these conditions would be expected to yield monoclinic paracetamol. There is a significant bias toward the production of orthorhombic paracetamol when crystallizations are carried out in the presence of Nylons or halogenated polymers. Rodríguez-Hornedo et al., J Pharm Sci. (2004) 93(2), 449-60, describe the use of surfactants sodium lauryl sulfate and sodium taurocholate on the crystallization of dihydrate carbamazepine.

Garekani et al., Int. J. of Pharmaceutics 208 (2000) 87, and literature cited therein, report some methods for modifying the crystal habit of paracetamol by crystallization in the presence of additives.

WO 05/115344 claims that a rapidly dissolving form of paracetamol is obtained after recrystallization in the presence of a crystallization modifier, which may be a polymer, or a protein such as albumin, papain, pepsin.

It has now been found that rapid nucleation of organic substances in solution may be induced, even at elevated temperatures, by an amphiphatic protein, especially a hydrophobin. It has also been found that such proteins alter the crystal growth behaviour of the organic substance during the crystallization process yielding unexpected crystal habits and crystal size distribution. Hydrophobins may be used as additives during or after crystallization, e.g. in order to control the morphology (stabilization of metastable polymorphs) and the size distribution of organic compounds such as bio-active substances, e.g. for cosmetical, biocidal, pharmaceutical or medical applications (such as cosmetical actives, active pharmaceutical ingredients [APIs], animal care products, agrochemicals, biocides, pigments, dyestuffs) or to stabilize certain polymorphs. The invention thus pertains to a process for modifying the morphology and/or polymorphism of an organic substance, which process comprises treating the solid substance, or a solution or dispersion thereof, with one or more amphiphilic proteins, as characterized in the appended claims.

The process is advantageously carried out using a solution or dispersion of the organic substance and/or solution or dispersion of the protein. The solution usually is one in a polar solvent, often an aqueous solvent such as water, lower alcohol (such as methanol, ethanol, propanol, isopropanol, butanol, isobutanol) or mixtures of water and lower alcohol, especially water.

One of the most important aspects of this invention is the modification of crystal properties of bio-active substances by employing the use hydrophobins during the crystallization process.

Most bio-active substances are found to be poorly soluble in water, and it is known that forming finer particles (micron and smaller) can improve their bio-availability or the dissolution rate due to their increased surface area.

Further described herein is thus a composition comprising an organic bio-active substance and an amphiphilic protein, especially a hydrophobin, such as the compositions obtainable in the process of the invention. The composition contains the organic bio-active substance preferably in fine grain form, with mean particle sizes, depending on the desired end use, e.g. ranging from 0.1 to 1000 micrometer, or in other cases as detectable by dynamic light scattering ranging e.g. from 5 to 5000 nm, especially 20 to 2000 nm. Such particles may be free flowing, dispersed in a liquid, or especially agglomerated or compacted. The amount of amphiphilic protein in the composition, e.g. in the solid particle composition, may cover a wide range depending un the end use and desired effect, ranging for example from about 0.0001 to about 10 %, often from 0.001 to about 2 %, especially 0.01 to about 1 %, each by weight of the final composition.

Thus, the present process relates to a modification which comprises a reduction of the crystallite size.

Also described herein is a process relating to a modification which comprises a change of crystal habit.

Further described herein is a process relating to a modification which comprises a change in crystal morphology.

Also described herein is a modification of an organic solid which comprises a combination of two or more of the following crystal properties:
(a) reduction of crystallite size; (b) a change of crystal habit and (c) a change in crystal morphology.

Amphiphilic proteins possess both hydrophobic and hydrophilic properties and can be termed as "nature's surfactants". A synonym to amphiphilic is "amphipathic".

Amphiphatic proteins can physically adsorb on the surface of a solid substance to form a surface possessing both hydrophobicity and hydrophilicity oriented in accordance with the wettability of the surface being treated.

If the surface is hydrophobic, the hydrophobic side of the coating is in contact with the hydrophobic surface being coated, and the outer surface of the coating is hydrophilic, thereby increasing the water wettability of the surface being coated.

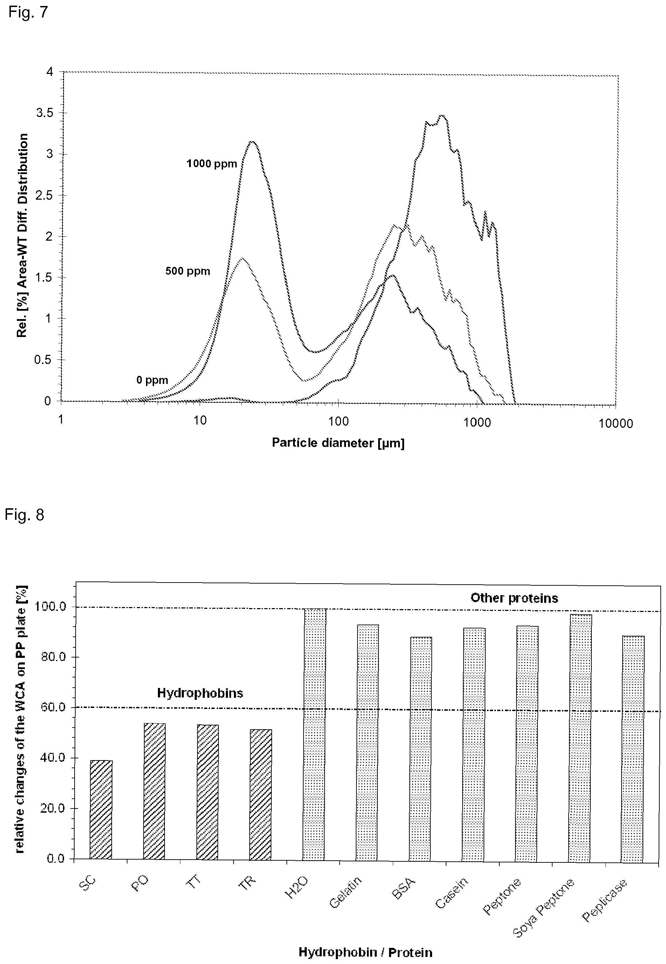

The surface active properties of proteins onto substrates can be assessed by interfacial tension measurements, characterization of oil-in-water emulsions and contact angles with water. The amphiphilic protein useful in the present invention is characterized by strongly lowering the contact angle of water (WCA) on a hydrophobic surface (e.g. the surface of a polyolefin or a Teflon® surface). Specifically, a 1% b.w. aqueous solution or dispersion of the amphiphilic protein useful in the present invention generallyshows a contact angle on a polypropylen surface (specifically: PP homopolymer type HC115M0, Borealis, melt flow rate = 4.0 g/10 min [230°C/2.16 kg]) which is lower than the contact angle observed for pure water by 30 degrees or more, more preferably 40 degrees or more, most preferably 45 degrees or more, and in some specific cases 50 degrees or more (see Fig. 8; all WCA measurements according to static sessile drop method).

Hydrophobins, discovered in 1991, are a class of small secreted cysteine-rich amphiphilic proteins present in fungi and fulfilling a broad spectrum of functions in fungal growth and development. Hydrophobins are among the most surface active molecules and self-assemble at any hydrophilic-hydrophobic interface into an amphiphilic film (Biochimica et Biophysica Acta - Reviews on Biomembranes - Volume 1469, Issue 2, 18 September 2000, Pages 79-86).

Hydrophobins are typically readily soluble in water. Spontaneous self-assembly of hydrophobins leads to the formation of an amphiphilic layer that remarkably reduces the surface tension of water. A suggested mechanism of the function for hydrophobins can be found in J. Biol. Chem., Vol. 282, Issue 39, 28733-28739, September 28, 2007: Monomers multimerize to dimers, two of which form a tetramer. The tetramer may split into two new dimers with hydrophobic surface areas aligned. These amphiphilic dimers precede the formation of amphiphilic monolayer on hydrophobic-hydrophilic interface. At high concentration, excess hydrophobin forms fibril structures.

Based on differences in hydropathy patterns and biophysical properties, hydrophobins are divided into two categories: class I and class II.
Examples of Class I:
   POH3 (PO) from *Pleurotus ostreatus*
   TT1 (TT) from *Talaromyces thermophilus*
   SC3 (SC) from *Schizophyllum commune*
Examples of Class II:
   HFBI & HFBII (TR) from *Trichoderma reesei*
   HCf-6 from *Cladosporium fulvum*
   CU *(Cerato-ulmin)*

Purification and isolation of hydrophobins are described in the following references:
WO-A-96/41882 describes hydrophobins from edible fungi.
WO-A-00/58342 relates to purification of hydrophobin-containing fusion proteins by phase extraction.
WO-A-01/57066 describes stabilization, solubilization and, related thereto, improved application of hydrophobins due to sulfite treatment.
WO-A-01/57076 describes purification of hydrophobin via adsorption to Teflon beads and elution by means of a detergent such as Tween at low temperatures.

United States Patent 7,147,912 cites hydrophobins belong to the most surface-active molecules. With a maximal lowering of the water surface tension from 72 to 24 mJ m⁻² at 50 microgram/ml, SC3 (Schizophyllum commune) is regarded as the most surface-active protein known. SC3 is a class 1 hydrophobin.

Further hydrophobins useful in the present invention, as well as sources and properties thereof, are described inter alia in WO 96/41882 (see passage from page 1, line 14, to page 7, line 20, and examples 1 to 5); WO 03/10331 (see passage from page 1, line 4, to page 5, line 20); or WO 06/103230 (see passage from page 3, paragraph 6, to page 12, 3^{rd} line from bottom of page).

Surprisingly it has been found that hydrophobins, also affect the morphological properties of organic substances and often give more advantageous compositions than when solvents, polymers or synthetic surfactants are used.

The present process often is carried out by
i) combining the solution or dispersion of the protein in a polar solvent, and a solution or dispersion of the organic substance in a polar solvent which is miscible with the solvent of the protein, or
ii) contacting the solution or dispersion of the organic substance in a polar solvent with a surface impregnated with the protein.

One embodiment comprises:
(a) dissolving or dispersing the amphiphilic protein in a solvent;
(b) dissolving the organic substance in a solvent that is miscible with the solvent of the protein;
(c) mixing (a) and (b)
(d) adjusting the physical environment of the mix to allow precipitation / crystallization of the organic substance to occur in the presence of the amphiphilic protein.

A second embodiment comprises:
(a) dissolving or dispersing the amphiphilic protein in a solvent;
(b) adding (a) to a reaction vessel in which an organic substance is to be formed by a chemical reaction;
(c) allow the chemical reaction to occur in the presence of the amphiphilic protein;
(d) adjust the physical environment of the mix to allow precipitation / crystallization of the organic substance to occur in the presence of the amphiphilic protein.

A third embodiment comprises:
(a) dissolving or dispersing the amphiphilic protein in a solvent;
(b) dissolving the organic substance in a solvent that is miscible with the solvent of the protein;
(c) mixing (a) and (b);
(d) wet milling the organic substance in the presence of the amphiphilic protein.

A fourth embodiment comprises:
the addition of "seeds" (stable nuclei) of the organic substance to the solution of the organic substance prior to or after the addition of the amphiphilic protein solution / dispersion.

A fifth embodiment comprises:
Combination of the amphiphilic protein(s) with other additives, especially those that can also influence the crystallization process of organic substances. Examples of such other additives are salts (such as sodium chloride, ammonium salts) or further polymers (especially soluble polymers including synthetic ones such as polyvinylpyrrolidone and natural ones such as gelatine). Within this definition small quantities of solvents are included.

Further described herein are the following aspects each of which may be combined with each other:
- A process for modifying the morphology and/or polymorphism of an organic substance, which process comprises treating the solid substance, or a solution or dispersion thereof, with one or more amphiphilic proteins.
- Said process wherein a 1% b.w. aqueous solution or dispersion of the amphiphilic protein(s) used is characterized by a contact angle on a polypropylene surface which is lower than the contact angle observable for pure water by 20 degrees or more.
- Said process which comprises precipitation, crystallization or solid-solid phase transition of the organic substance.
- Said process which comprises combining the solution or dispersion of the protein in a polar solvent, and a solution or dispersion of the organic substance in a polar solvent which is miscible with the solvent of the protein; or contacting the solution or dispersion of the organic substance in a polar solvent with a surface impregnated with the protein.
- Said process which comprises wet milling the organic substance in the presence of the amphiphilic protein(s).
- Said process which comprises the addition of crystal seeds of the organic substance to the solution of the organic substance prior to, or after, the addition of the amphiphilic protein(s).
- Said process wherein a solution contains a further additive such as a salt and/or a polymer.
- Said process wherein the organic substance is an organic compound, which is solid at 0° Celsius and soluble, partially soluble or dispersable in the polar solvent, for example as a colloid.
- Said process wherein the organic substance is an organic compound from the molecular weight range 80 to 1000, especially 100 to 500 g/mol, which is preferably selected from bio-active compounds such as drugs, pharmaceutical and cosmetical ingredients, pesticides, and fungicides.
- Said process for the reduction of the crystallite size, increase of amorphous portion, change of crystal habit and/or change of crystal polymorphy.
- Said process wherein the protein is a hydrophobin, such as a class II hydrophobin or especially a class I hydrophobin.
- Said process wherein precipitation or crystallization of the organic substance is induced by combining a solution or dispersion thereof with the solution or dispersion of the amphiphilic protein.
- The use of an amphiphilic protein, especially as characterized in any of the embodiments above, for modifying the morphology and/or polymorphism of an organic substance.
- A composition comprising a solid organic substance, especially as defined above or listed forther below, and an amphiphilic protein, especially as characterized in any of the embodiments above.
- Said composition comprising the solid organic substance in the form of fine grain particles, e.g. of average size 0.1 to 1000 micrometer, or 5 to 5000 nm.

Also described herein is the use of an amphiphilic protein, especially as characterized by lowering of the contact angle as described above, especially a hydrophobin as described above, for modifying the morphology and/or polymorphism of an organic substance; as well as a composition comprising a solid organic substance, especially an organic compound, which is solid at 0° Celsius and soluble, partially soluble or dispersable in the polar solvent, for example as a colloid, and/or an organic compound from the molecular weight range 80 to 1000, especially 100 to 500 g/mol, which is preferably selected from bio-active compounds such as drugs, pharmaceutical and cosmetical ingredients, pesticides, and fungicides, in combination with the amphiphilic protein. Said composition preferably comprises the solid organic substance in the form of fine grain particles.

Examples for bio-active organic substances whose solid form may be modified by the present process include the following ones:
Pharmaceutical ingredients (APIs): acarbose, acetylsalicylic acid, alfuzosin, aliskiren, ambrisentan, amlodipine, amoxicillin, anastrozole, apixaban, aprepitant, aripiprazole, atazanavir, atenolol, atomoxetine, atorvastatin, azithromycin, bazedoxifene, benazepril, bicalutamide, bisacodyl, budesonide, butylscopolamine, candesartan, capecitabine, carbamazepine, carisbamate, carvedilol, casopitant, celecoxib, cetirizine, chloroquine, cinacalcet, ciprofloxacin, clavulanic acid, clodronate, clonidine, clopidogrel, cyproterone acetate, dapoxetine, darunavir, dasatinib, deferasirox, dextromethorphan, diclofenac, dienogest, dipyridamole, docetaxel, donepezil, drospirenone, duloxetine, efavirenz, eletriptan, enalapril, entacapone, entecavir, enzastaurin, erlotinib, esomeprazole, eszopiclone, ethinylestradiol, etoricoxib, etravirine, everolismus, exemestane, fexofenadine, finasteride, fluoxetine, fluticasone, fluticasone propionate, fluvastatin, formoterol, ganciclovir, gefitinib, glimepiride, hydrocodone, ibandronic acid, ibuprofen, indinavir, ipratropium, irbesartan, lamotrigine, lansoprazole. lapatinib, letrozole, levonorgestrel, linezoild, lisinopril, losartan, maraviroc, meloxlcam, metformin, methylphenidate, metoprolol, moxidectin, mycophenolic acid, naproxen, nateglinide, nevirapine, nicorandil, nifedipine, nilotinib, olanzapine, omeprazole, orlistat, oseltamivir, oxaliplatin, oxcarbazepine, paliperidone, pantoprazole, paracetamol, paroxetine, pioglitazone, pramipexole, pravastatin, pregabalin, quetiapine, rabeprazole, raloxifene, ramipril, reboxetine, risedronate sodium, rivaroxaban, rivastigmine, rizatriptan, rosiglitazone, ruboxistaurin, salmeterol, sildenafil citrate, simvastatin, sirolimus, sitagliptin, sorafenib, sumatriptan, sunitinib, surinabant, tadalafil, tamsulosin, tapentadol, telbivudine, telmisartan, terbinafine hydrochloride, teriflunomide, tiotropium, tolterodine, topiramate, vabicaserin hydrochloride, valaciclovir, valganciclovir, valsartan, vandetanib, vardenafil, varenicline, venlafaxine, vildagliptin, voriconazole, warfarin, ziprasidone, zolmitriptan, zolpidem.

Further drugs: acepromazine, amoxicillin, ampicillin, apramycin, benazepril, betamethasone, buscopan, carprofen, cefapirin, clenbuterol, clindamycin, cloxacillin, cyclosporine A, cyromazine, deracoxib, dichlorvos, dicyclanil, difloxacin, enrofloxacin, etodolac, fenbendazole, framycetin, furosemide, griseofulvin, hetacillin, hygromycin, imidacloprid, levamisole, levothyroxine, lufenuron, meloxicam, milbemycin oxime, monensin, moxidectin, narasin, nicarbazin, nitenpyram, oleandomycin, oxfendazole, oxyclozanide, paramectin, paromomycin, permethrin, phenylbutazone, praziquantel, procaine benzylpenicillin, procaine penicillin, pyrantel pamoate, spinosad, sulphadiazine, thiamethoxam, tiamulin, triamcinolone, triclabendazole, trimethoprim, tylosin.

Agrochemicals such as pesticides and fungicides: abamectin, brodifacoum, cyromazine, emamectin, fenoxycarb, pirimicarb, pymetrozine, thiamethoxam.

### Cosmetic ingredients such as

UV filter substances (e.g. (+/-)-1,7,7-trimethyl-3-[(4-methylphenyl)methylene]bicyclo-[2.2.1]heptan-2-one; 1,7,7-trimethyl-3-(phenylmethylene)bicyclo[2.2.1]heptan-2-one; (2-Hydroxy-4-methoxyphenyl)(4-methylphenyl)methanone; 2,4-dihydroxybenzophenone; 2,2',4,4'-tetrahydroxybenzophenone; 2-Hydroxy-4-methoxy benzophenone; 2-Hydroxy-4-methoxy benzophenone-5-sulfonic acid; 2,2'-dihydroxy-4,4'-dimethoxybenzophenone; 2,2'-Dihydroxy-4-methoxybenzophenone; Alpha-(2-oxoborn-3-ylidene)toluene-4-sulphonic acid and its salts; 1-[4-(1,1-dimethylethyl)phenyl]-3-(4-methoxyphenyl)propane-1,3-dione; Methyl N,N,N-trimethyl-4-[(4,7,7-trimethyl-3-oxobicyclo[2,2,1]hept-2-ylidene)methyl]anilinium sulphate; 3,3,5-Trimethyl cyclohexyl-2-hydroxy benzoate; Isopentyl p-methoxycinnamate; Menthyl-o-aminobenzoate; Menthyl salicylate; 2-Ethylhexyl 2-cyano,3,3-diphenylacrylate; 2- ethylhexyl 4-(dimethylamino)benzoate; 2- ethylhexyl 4- methoxycinnamate; 2- ethylhexyl salicylate; Benzoic acid, 4, 4', 4"- (1,3,5- triazine- 2,4,6- triyltriimino)tris-, tris(2-ethylhexyl)ester; 2,4,6-Trianilino-(p-carbo-2'-ethylhexyl-1'-oxi)-1,3,5-triazine; 4- aminobenzoic acid; Benzoic acid, 4-amino-, ethyl ester, polymer with oxirane; 2- phenyl- 1 H- benzimidazole- 5- sulphonic acid; 2-Propenamide, N-[[4-[(4,7,7-trimethyl-3-oxobicyclo[2.2.1]hept-2-ylidene)methyl] phenyl] methyl]-, homopolymer; Triethanolamine salicylate; 3, 3'- (1, 4- phenylenedimethylene)bis[7, 7- dimethyl- 2- oxo- bicyclo[2.2.1]heptane-1- methanesulfonic acid]; 2,2'-Methylene-bis-[6-(2H-benzotriazol-2-yl)-4-(1,1,3,3-tetramethylbutyl)-phenol]; 2,4-bis{[4-(2-ethylhexyloxy)-2-hydroxy]-phenyl}-6-(4-methoxyphenyl)-(1,3,5)-triazine; 1 H-Benzimidazole-4,6-disulfonic acid, 2,2'-(1,4-phenylene)bis-, disodium salt ; Benzoic acid, 4,4'-[[6-[[4-[[(1,1-dimethylethyl)amino]carbonyl]phenyl]amino]1,3,5-triazine-2,4-diyl]diimino]bis-, bis(2-ethylhexyl)ester; Phenol, 2-(2H-benzotriazol-2-yl)-4-methyl-6-[2-methyl-3-[1,3,3,3-tetramethyl-1-[(trimethylsilyl)oxy]disiloxanyl]propyl]-; Dimethicodiethylbezalmalonate; Benzenesulfonic acid, 3-(2H-benzotriazol-2-yl)-4-hydroxy-5-(1-methylpropyl)-, monosodium salt; Benzoic acid, 2-[4-(diethylamino)-2-hydroxybenzoyl]-, hexyl ester; 1-Dodecanaminium, N-[3-[[4-(dimethylamino)benzoyl]amino]propyl]N,N-dimethyl-, salt with 4-methylbenzenesulfonic acid (1:1); 1-Propanaminium, N,N,N-trimethyl-3-[(1-oxo-3-phenyl-2-propenyl)amino]-, chloride; 1 H-Benzimidazole-4,6-disulfonic acid, 2,2'-(1,4-phenylene)bis-1,3,5-Triazine, 2,4,6-tris(4-methoxyphenyl)-; 1,3,5-Triazine, 2,4,6-tris[4-[(2-ethylhexyl)oxy]phenyl]-; 1-Propanaminium, 3-[[3-[3-(2H-benzotriazol-2-yl)-5-(1,1-dimethylethyl)-4-hydroxyphenyl]-1-oxopropyl]amino]-N,N-diethyl-N-methyl-, methyl sulfate (salt); 2-Propenoic acid, 3-(1 H-imidazol-4-yl)-; Benzoic acid, 2-hydroxy-, [4-(1-methylethyl)phenyl]methyl ester; 1,2,3-Propanetriol, 1-(4-aminobenzoate); Benzeneacetic acid, 3,4-dimethoxy-a-oxo-; 2-Propenoic acid, 2-cyano-3,3-diphenyl-, ethyl ester; Anthralinic acid, p-menth-3-yl ester; 2,2'-bis(1,4-phenylene)-1H-benzimidazole-4,6-disulphonic acid mono sodium salt or Disodium phenyl dibenzimidazole tetrasulfonate or Heliopan AP);
active ingredients against insects (repellents; such as diethyl toluamide (DEET); or other common repellents as may be found in "Pflegekosmetik" (W. Raab and U. Kindl, Gustav-Fischer-Verlag Stuttgart/New York, 1991), page 161);
active ingredients having a keratoplastic effect (e.g. benzoyl peroxide, retinoic acid, colloidal sulfur and resorcinol);
antimicrobial agents (for example triclosan or quaternary ammonium compounds); antioxidants.

Organic pigments such as phthalocyanines, azos, etc
Organic dyes such as solvent dyes, direct dyes etc

The invention is not limited to the above list of organic substances.

The following test methods and examples are for illustrative purposes. Room temperature (r.t. or RT) depicts a temperature in the range 20-25°C; over night denotes a time period in the range 12-16 hours. Percentages are by weight, temperatures by degrees Celsius (centigrade) unless otherwise indicated.

### Abbreviations used in the examples or elsewhere:

- ACN: acetonitrile
- API: active pharmaceutic ingredient
- BSA: bovine serum albumine (Fluka)
- IPA: isopropanol
- PO: class I hydrophobin from *Pleurotus ostreatus,*
- PP: polypropylene
- RT: room temperature
- SC: class I hydrophobin from *Schizophyllum commune,*
- TR: class II hydrophobin from *Trichoderma reesei,*
- TT: class I hydrophobin from *Talaromyces thermophilus,*
- WCA: water contact angle on PP sheet (static sessile drop method)
- w/w: parts or percentage by weight relative to total weight.

### Reparation of Hydrophobins

Hydrophobin solution: Aqueous solutions are used containing 10 mg protein/ml solution. Hydrophobins are produced recombinantly in *E. coli*; these are
class I:
   PO from *Pleurotus ostreatus,*
   TT from *Talaromyces thermophilus,*
   SC from *Schizophyllum commune,*
and class II:
   TR from *Trichoderma reesei.* Hydrophobin from *Trichoderma reesei* can also be obtained by submerged fermentation of the wild type and work up.

### Application Examples

### Example 1: Crystallisation of Paracetamol in presence of Hydrophobins

The hydrophobin solution is prepared by solubilization of 10 mg of lyophilized powder (protein content at least 60-80%) in 1 mL pure water. Insoluble residues are removed by centrifugation.

15 g of API (Paracetamol, Sigma) are dissolved in 150 mL pure water. Aliquots of 10 mL of the resulting solution are transferred into 15 mL sealable polypropylene tubes that have been pre-heated to 95°C in order to prevent the onset of crystallization. To ensure uniform temperature, the tubes are shaken for 15-30 min at 95° and 750 rpm on a thermomixer *(Eppendorf Thermomixer Comfort),* then 100 µL of the hydrophobin solution is added under continued mixing at 95°C; final protein concentration in the sample is 100 ppm. After 1-2 min the tubes are withdrawn from the thermomixer to allow cooling without shaking at RT. Control samples are treated in the same way except that 100 µL of pure water are added instead of the hydrophobin solution, or no API is present. Crystallization behaviour is assessed visually; the results are compiled in the following table.

**Table 1**

| Sample | protein | API | after 0 min | < 5 min | 15 min | > 20 h |
|---|---|---|---|---|---|---|
| control | none | yes | streaks | clear | clear | crystals 1-2 mm |
| invention | SC | yes | streaks | clear | cryst. 0.1-0.2 mm | crystals 0.5 mm |
| control | SC | - | clear | | | |
| invention | TR | yes | streaks | clear | clear | crystals 0.5 mm |
| control | TR | - | clear | | | |
| invention | PO | yes | clouding | few crystals 0.2-0.5 mm | cryst. 0.2-0.5 mm | crystals 0.5-1 mm |
| control | PO | - | clear | | | |
| invention | TT | yes | clouding | few cryst. 0.1 mm | crystals 0.1 mm | crystals 0.5-1 mm |
| control | TT | - | clear | | | |

"Streaks" denotes observation of initial concentration streaks without clouding.
Clouding is effected by the formation of small paracetamol crystals. The controls in absence of API show that no denaturation of the protein occurs (no clouding).

All crystals formed are dispersable.

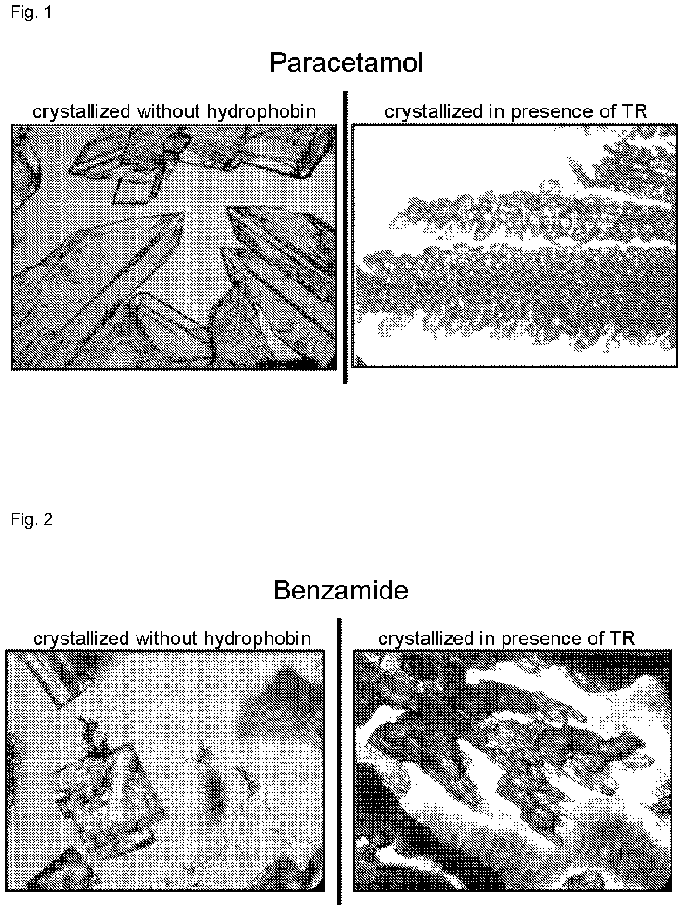

A few minutes (<5) after injection, 5 mL of the mother liquor described above are poured into dishes (0 5 cm) in order to allow crystallization by evaporation. The dried crystals obtained after about 20 h are visually inspected; similar crystal forms and sizes are obtained for all class I hydrophobins, while crystallization in presence of TR yields large, dendrite-like crystals (Fig. 1).

### Example 2: Crystallisation of benzamide in presence of SC, PO, TT or TR

15 g of benzamide (Fluka) are dissolved in 150 mL pure water. Aliquots of 10 mL of the resulting solution are transferred into 15 mL sealable polypropylene tubes that have been pre-heated to 95°C in order to prevent the onset of crystallization. To assure uniform temperature, the tubes are shaken for 15-30 min at 95° and 750 rpm on a thermomixer (*Eppendorf Thermomixer Comfort),* then 100 µL of the hydrophobin solution (prepared according to the method given in example 1) is added (the tubes are still shaken to allow homogeneous mixing and tempering). After 1-2 min the tubes are withdrawn from the thermomixer to allow cooling without shaking at RT. Control samples are treated in the same way except that 100 µL of pure water are added instead of the hydrophobin solution, or no API is present.
The results of a visual assessment after the hydrophobin injection (final concentration in the sample is 100 ppm) are compiled in the following table.

**Table 2**

| protein | after 0 min | < 5 min | 15 min | > 20 h* |
|---|---|---|---|---|
| none | streaks | clear | clear | crystals 1-2 mm |
| SC | clouding | few crystals 0.1-0.2 mm | crystals 0.1-0.2 mm | crystals 0.5-1 mm |
| TR | streaks | clear | few crystals 0.1 mm | crystals 0.2-0.5 mm |
| PO | clouding | few crystals 0.2-0.5 mm | crystals 0.2-0.5 mm | crystals 0.5-1 mm |
| TT | clouding | few crystals 0.1 mm | crystals 0.1 mm | crystals 0.5-1 mm |

"Streaks" denotes observation of initial concentration streaks without clouding.
Clouding is due to the formation of small paracetamol crystals and not to the denaturation of added protein). * all crystals are dispersable

A few minutes (<5) after injection, 5 mL of the mother liquor are poured into dishes (0 5 cm) in order to allow crystallization by evaporation. The dried crystals obtained after about 20 h are visually inspected; similar crystal forms and sizes are obtained for all class I hydrophobins, while crystallization in presence of TR yields bundled, elongated crystals (Fig. 2).

### Example 3: Crystallisation and redispersion of Venlafaxine

15 g of Venlafaxine (Ciba) are dissolved in 150 mL IPA. Aliquots of 10 mL of the resulting solution are transferred into 15 mL sealable polypropylene tubes that have been pre-heated to 95°C. To assure uniform temperature, the tubes are agitated for 15-30 min at 80° and 750 rpm on a thermomixer (*Eppendorf Thermomixer Comfort),* then 100 µL of the protein solution (prepared according to the method given in example 1) is added. Control samples are treated in the same way except that 100 µL of pure water are added instead of the hydrophobin solution, or no API is present.

The results of a visual assessment after the hydrophobin injection (final concentration in the sample is 100 ppm) are compiled in the following table.

**Table 3: Visual assessment of crystallization and dispersability (RT) of the crystals**

| Sample Protein | after 0 min | < 5min | 20 min | 120 min (RT) | dispersability |
|---|---|---|---|---|---|
| control none | streaks | clear | clear | crystals | poor |
| invention SC | streaks | clear | clear | crystals | best |
| invention TR | streaks | clear | clear | crystals | good |
| invention PO | clouding | crystals 0.1 mm | crystals 0.2 mm | crystals | poor |
| invention TT | streaks | clouding | crystals 0.2 mm | crystals | poor |

Clouding is interpreted as the formation of small API crystals.
At room temperature, crystallization is far advanced and the tube is filled with a bulky mass of crystal aggregates which does not allow determination of crystal size.

The mother liquor crystallizes completely over night, with crystals adhering together covered by the solvent. Vigorous shaking (Vortex) for 10-15 sec is accompanied by different degrees of re-suspension: pouring the suspensions into dishes results in complete emptying of the tubes for the samples containing SC and TR, while for samples containing PO, TT, and the control sample, predominantly the remaining mother liquor is poured out.
Best dispersability is obtained in presence of SC or TR .

For Venlafaxine, crystallization temperature and the dispersability of the crystals obtained is affected by the protein.

### Example 4: Crystallisation of Atorvastain

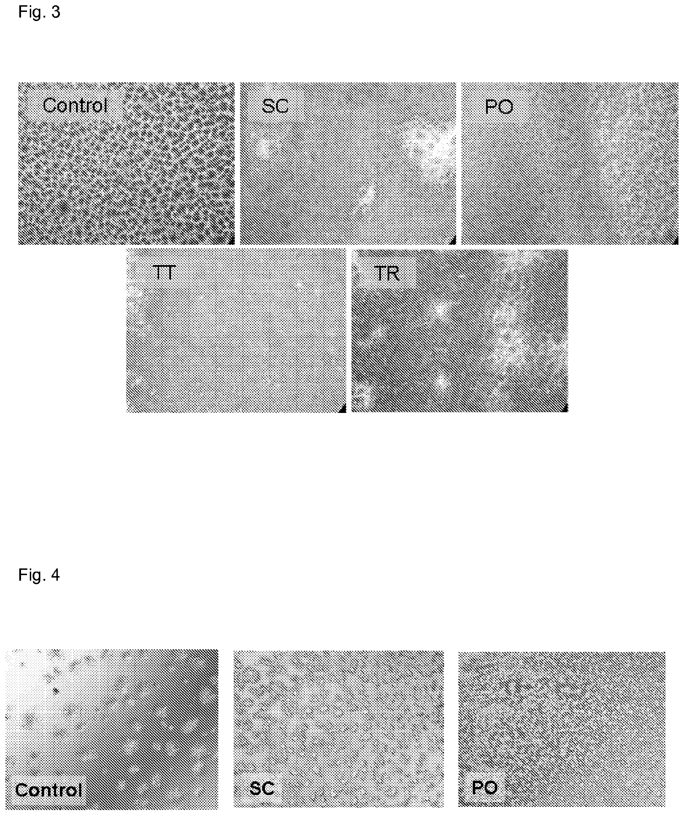

a) 15 g of Atorvastatin (Ciba) are dissolved in 75 mL pure water and 75 mL acetonitrile by heating to 50°C and stirring for 2 h. The resulting solution is filtered (0.45 µm). Aliquots of 10 mL are transferred into 15 mL sealable polypropylene tubes that have been pre-heated to 50°C in order to prevent the onset of crystallization. To assure uniform temperature, the tubes are shaken for 15-30 min at 50° and 750 rpm on a thermomixer (*Eppendorf Thermomixer Comfort),* then 100 µL of the protein solution (prepared according to the method given in example 1) is added (final concentration: 100 ppm). Control samples are treated in the same way except that 100 µL of pure water are added instead of the hydrophobin solution, or no API is present.
   1 mL of each sample is poured into dishes (∅ 5 cm) in order to allow crystallization by evaporation. The dried crystals (-20 h later) are microscopically inspected. Addition of an hydrophobin leads to more evenly crystal growth (fig. 3).
b) In a further set of tests, object slides are coated with protein by incubation in a solution as prepared according to the method given in example 1 (100 µg protein/mL) over night. The object slides then are washed and air dried before 50 µL of Atorvastatin solution as described above is coated on the slide. As is shown in Figure 4, the crystallisation by evaporation on hydrophobin coated glass surface is accompanied by a more regular crystal growth, especially after coating with PO or SC.

### Example 5: Crystallisation of paracetamol or benzamide, varying protein concentration

All solutions and aliquots are prepared as described in example 1 and 2 except that 500 µL of a solution with different amounts of TR (to adjust final concentrations of 100 / 250 / 500 / 750 /1000 ppm) are added. The TR stock solution is prepared by solubilizing 30 mg lyophilized TR in 1.5 mL pure water. The injection of the TR solution is not accompanied by turbidity, but for concentrations over 250 ppm the liquid starts getting slightly clouded after less than 5 min. At that time, 5 mL of each sample is poured into a dish to allow crystallization by evaporation.

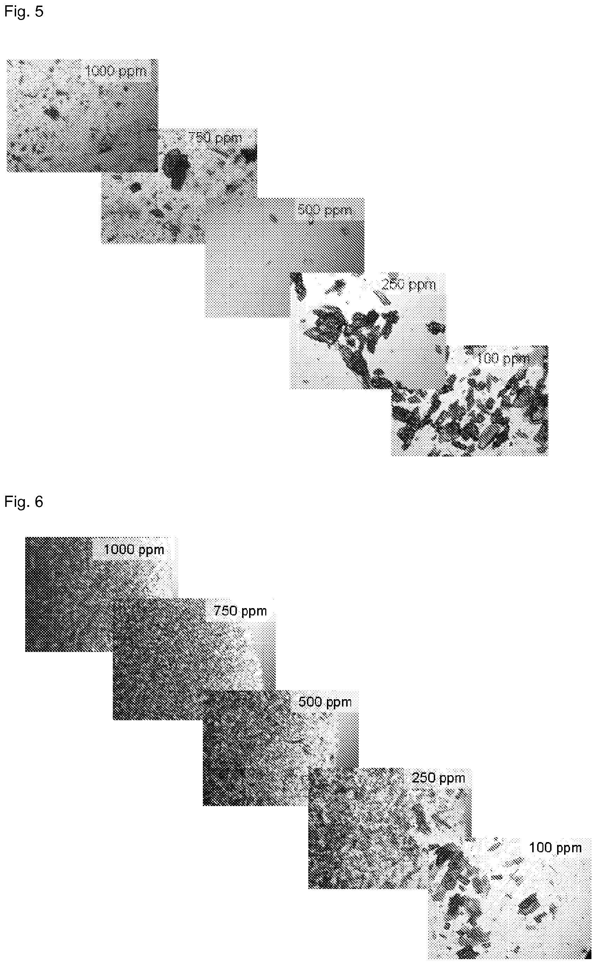

Higher concentrations of TR are clearly characterized by a declining size of the crystal and more stable dispersion of the crystals in the mother liquor for both paracetamol and benzamide (Fig. 5 + 6).

For the paracetamol samples, crystal size fractions (weight by the particle's surface) are determined by the single-particle optical sensing (SPOS) method (*AccuSizer 780*/*A, Particle Sizing Systems*). Without hydrophobin, the crystal size predominantly ranges from 0.1 to 2 mm; the addition of TR leads to the formation of a class of smaller particles in the size range of 5-50 µm. Further, the presence of TR significantly reduces the size of the most abundant particles of the "large" fraction (see fig. 7). The hydrophobin-induced shift between the two predominant size fractions is compiled in table 5.

**Table 5: Percentage (w/w) of paracetamol crystal size fractions**

| Concentration of TR | Fraction 1 (5-50 µm) | Fraction 2 (0.1-2 mm) |
|---|---|---|
| 0 ppm | 1.2 % | 98.8 % |
| 500 ppm | 34.8 % | 65.2 % |
| 1000 ppm | 57.1 % | 42.9 % |

### Brief description of Figures:

**Fig. 1****:** Paracetamol (50-times magnified) after crystallization in presence of TR as compared to the hydrophobin-free control reveals a distinct modification of crystal habit.
**Fig. 2****:** Benzamide (50-times magnified) after crystallization in presence of TR as compared with the hydrophobin-free control reveals a distinct modification of crystal habit.
**Fig. 3****:** Atorvastatin solutes after crystallization by evaporation (magnification: 50-times); presence of 100 ppm of a hydrophobin (SC, PO, TT, TR) results in a more evenly crystal growth compared to the hydrophobin-free control.
**Fig. 4****:** Atorvastatin solutes after crystallization by evaporation on object slides (50-times magnified): Coating with PO or SC results in a more evenly crystal growth compared to the non-coated control.
**Fig. 5** (Magnification 50x): Paracetamol after crystallization in presence of increasing TR concentrations (100 - 1000 ppm) results in declining average crystal size.
**Fig. 6** (Magnification 50x): Benzamide after crystallization in presence of increasing TR concentrations (100 - 1000 ppm) results in declining average crystal size.
**Fig. 7****:** Surface weight particle size distribution of paracetamol crystals in absence or presence of TR; protein additon affects the size distribution and leads to smaller particles.
**Fig. 8****:** Relative change of water contact angle of a 1 % b.w. protein solution on a polypropylene plate (Borealis HC115MO) compared to pure water, demonstrating high amphiphilicity of hydrophobins.
**Fig. 9****:** Schematic steps in a typical crystallization process.

## Claims

1. Process for modifying the morphology and/or polymorphism of an organic substance, wherein the process is for the reduction of crystallite size, which process comprises treating the solid substance, or a solution or dispersion thereof, with one or more amphiphilic proteins, which amphiphilic protein is a hydrophobin, such as a class II hydrophobin or especially a class I hydrophobin, and wherein the process comprises
i) combining the solution or dispersion of the protein in a polar solvent, and a solution or dispersion of the organic substance in a polar solvent which is miscible with the solvent of the protein, or
ii) contacting the solution or dispersion of the organic substance in a polar solvent with a surface impregnated with the protein.

2. Process according to claim 1 which comprises wet milling the organic substance in the presence of the amphiphilic protein(s).

3. Process according to any of the foregoing claims which comprises the addition of crystal seeds of the organic substance to the solution of the organic substance prior to, or after, the addition of the amphiphilic protein(s).

4. Process according to any of the foregoing claims wherein the solution contains a further additive such as a salt and/or a polymer.

5. Process according to any of the foregoing claims, wherein the organic substance is an organic compound, which is solid at 0° Celsius and soluble, partially soluble or dispersable in the polar solvent, for example as a colloid.

6. Process according to any of the foregoing claims, wherein the organic substance is an organic compound from the molecular weight range 80 to 1000, especially 100 to 500 g/mol, which is preferably selected from bio-active compounds such as drugs, pharmaceutical and cosmetical ingredients, pesticides, and fungicides.

7. Process according to any of the foregoing claims, wherein precipitation or crystallization of the organic substance is induced by combining a solution or dispersion thereof with the solution or dispersion of the amphiphilic protein.

## Patentansprüche

1. Verfahren zur Modifizierung der Morphologie und/oder der Polymorphie einer organischen Substanz, wobei das Verfahren zur Reduktion der Kristallitgröße ist, wobei das Verfahren die Behandlung der festen Substanz oder einer Lösung oder Dispersion davon mit einem oder mehreren amphiphilen Protein/en umfasst, wobei das amphiphile Protein ein Hydrophobin ist, wie etwa ein Klasse II Hydrophobin oder insbesondere ein Klasse I Hydrophobin und wobei das Verfahren
i) die Kombination der Lösung oder Dispersion des Proteins in einem polaren Lösungsmittel und einer Lösung oder Dispersion der organischen Substanz in einem polaren Lösungsmittel, das mit dem Lösungsmittel des Proteins mischbar ist, oder
ii) das Inkontaktbringen der Lösung oder Dispersion der organischen Substanz in einem polaren Lösungsmittel mit einer Oberfläche, die mit dem Protein imprägniert ist,
umfasst.

2. Verfahren nach Anspruch 1, das die Nassvermahlung der organischen Substanz in Anwesenheit des/der amphiphilen Proteins/e umfasst.

3. Verfahren nach einem der vorhergehenden Ansprüche, das die Zugabe von Impfkristallen der organischen Substanz zur Lösung der organischen Substanz vor oder nach der Zugabe des/der amphiphilen Proteins/e umfasst.

4. Verfahren nach einem der vorhergehenden Ansprüche, wobei die Lösung ein weiteres Additiv umfasst, wie etwa ein Salz und/oder ein Polymer.

5. Verfahren nach einem der vorhergehenden Ansprüche, wobei die organische Substanz eine organische Verbindung ist, die bei 0°C fest ist und löslich, teilweise löslich oder dispergierbar in dem polaren Lösungsmittel, beispielsweise als ein Kolloid, ist.

6. Verfahren nach einem der vorhergehenden Ansprüche, wobei die organische Substanz eine organische Verbindung des Molekulargewichtbereichs von 80 bis 1000, insbesondere von 100 bis 500 g/mol ist, die bevorzugt aus bioaktiven Verbindungen wie etwa Arzneimitteln, pharmazeutischen und kosmetischen Inhaltsstoffen, Pestiziden und Fungiziden ausgewählt ist.

7. Verfahren nach einem der vorhergehenden Ansprüche, wobei die Präzipitation oder die Kristallisation der organischen Substanz durch die Kombination einer Lösung oder Dispersion davon mit der Lösung oder Dispersion des amphiphilen Proteins induziert wird.

## Revendications

1. Procédé pour modifier la morphologie et/ou le polymorphisme d'une substance organique, lequel procédé a pour but la réduction de la taille des cristallites, lequel procédé comprend le traitement de la substance solide, ou d'une solution ou dispersion de celle-ci, avec une ou plusieurs protéines amphiphiles, laquelle protéine amphiphile est une hydrophobine, telle qu'une hydrophobine de classe II ou en particulier une hydrophobine de classe I, et lequel procédé comprend
i) la combinaison de la solution ou dispersion de la protéine dans un solvant polaire, et d'une solution ou dispersion de la substance organique dans un solvant polaire qui est miscible avec le solvant de la protéine, ou
ii) la mise en contact de la solution ou dispersion de la substance organique dans un solvant polaire avec une surface imprégnée de la protéine.

2. Procédé selon la revendication 1, qui comprend le broyage à l'état humide de la substance organique en présence de la ou des protéines amphiphiles.

3. Procédé selon l'une quelconque des revendications précédentes, qui comprend l'addition de germes cristallins de la substance organique à la solution de la substance organique, avant ou après l'addition de la ou des protéines amphiphiles.

4. Procédé selon l'une quelconque des revendications précédentes, dans lequel la solution contient un autre additif tel qu'un sel et/ou un polymère.

5. Procédé selon l'une quelconque des revendications précédentes, dans lequel la substance organique est un composé organique qui est solide à 0 °C et soluble, partiellement soluble ou dispersible dans le solvant polaire, par exemple sous la forme d'un colloïde.

6. Procédé selon l'une quelconque des revendications précédentes, dans lequel la substance organique est un composé organique ayant une masse moléculaire située dans la plage allant de 80 à 1000, en particulier de 100 à 500 g/mol, qui est de préférence choisi parmi les composés bioactifs tels que les médicaments, les ingrédients pharmaceutiques et cosmétiques, les pesticides, et les fongicides.

7. Procédé selon l'une quelconque des revendications précédentes, dans lequel la précipitation ou la cristallisation de la substance organique est induite par combinaison d'une solution ou dispersion de celle-ci avec la solution ou dispersion de la protéine amphiphile.
